# EUROPEAN PATENT APPLICATION

(11) **EP 2 363 510 A1**
(43) Date of publication of application: **07.09.2011**
(21) Application number: 10154838.6
(22) Date of filing: 26.02.2010
(51) Int. Cl.: C23C 14/02, C23C 14/06, A61L 27/30, A61L 31/08, C23C 14/32, C23C 14/35

(54) **Surface coatings for medical implants**

(71) Applicant: IMC Infomusic Consultants GmbH, 1010 Wien (AT)
(72) Inventor: Lammer, Johannes, 6535 Roveredo (CH)
(74) Representative: Köhler-Pavlik, Johann

(57) **Abstract**

The present invention relates to a deformable single layer ceramic coating comprising a multiplicity of cross-sectional zones, wherein the zone adjacent to the substrate is an adhesion zone composed of a solid transition metal of group IV B, V B or VI B of the periodic table of the elements or a combination thereof and one zone comprises double nitride (M₂N) , said one zone being 0.1 to 25 nm thick; wherein M is a solid transition metal of group IV B, V B or VI B of the periodic table of the elements or a solid solution thereof.

## Description

The present invention relates to surface coatings usable for medical implants and micromechanical devices.

Medical implants such as orthopaedic or cardiovascular implants, are usually metallic alloys, ceramics and composites formed from biocompatible polymers and various reinforcing materials.

Metals and metal alloys such as stainless steel, titanium and titanium alloys have been used successfully for many years as implant materials, particularly for orthopaedic applications. These materials have the requisite strength characteristics needed for such implants but are susceptible to fretting, wear and corrosion in the body unless treated to reduce these effects. Particulates produced by joint articulation processes or micro motion between assembled devices tend to cause accelerated wear of prosthetic joints and trauma devices.

Further, concern has been raised about potential abrasion between implant metals and adjacent bone and bone cement. This abrasion creates particulates, which are associated with adverse cellular response, including bone cell death and eventual loosening of the implant and subsequent revision.

A major problem of implants known in the art, in particular of deformable implants such as stents, is that the biocompatible coating of such implants, when deformed or subjected to physical stress, tends to chip off. In order to avoid this problem several solutions are suggested in the art.

WO 2006/067031, for instance, relates to implants being coated with a non-porous barrier layer consisting essentially of an essentially stoechiometric titanium nitride layer. The coating described therein suffers several shortcomings. In particular the question of adhesion is object of multiple statistic variations, thus necessitating extensive quality management tools, increasing by this unduly the cost of production. The multilayer coating according to WO 2006/067031 contains an elevated number of interfaces between the functional layers, which increases the probability of failure (scaling) of the coating. The main drawback of these coatings resides in the fact that it contains six or even 12 interfaces.

It is an object of the present invention to provide methods and means to overcome the drawbacks of the state of the art. the solid transition metal of the adhesion zone is zirconium, titanium, chromium or a combination thereof.

The present invention relates to a single layer ceramic coating comprising a multiplicity of cross-sectional zones, wherein the zone adjacent to the substrate is an adhesion zone composed of a solid or a combination thereof and one zone comprises double nitride (M₂N), said one zone being 0.1 to 25 nm thick, wherein M is a solid transition metal of group IV B, V B or VI B of the periodic table of the elements.

It surprisingly turned out that a coating comprising M₂N on a deformable substrate is much more stable against chipping off of the coating when subjected to extreme strain and stress. Such a coating can be obtained, for instance, with the method described below.

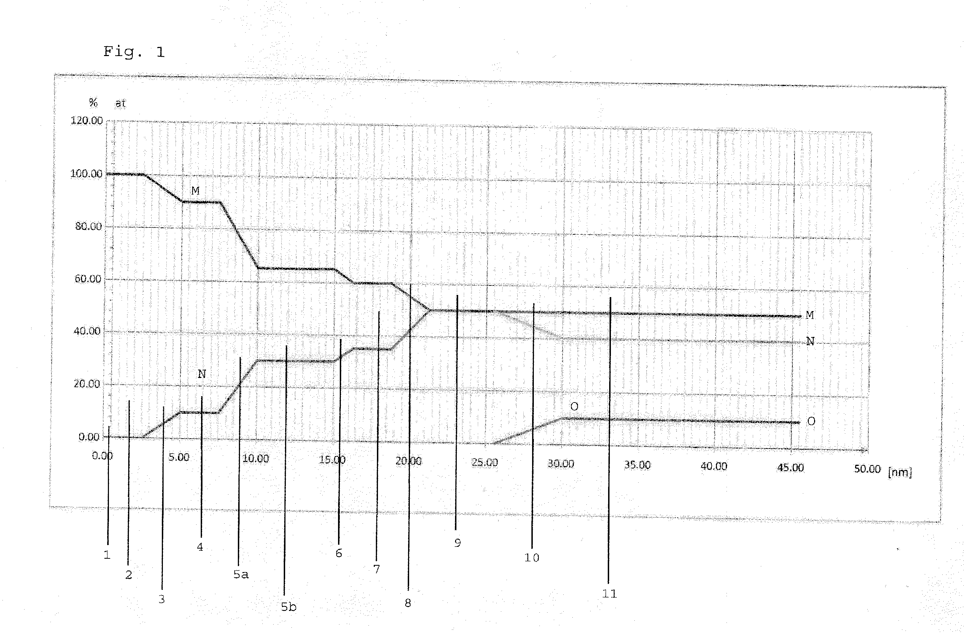

The various cross-sectional zones of the coating layer of the present invention are stoichiometrically non-homogeneous. Furthermore the areas between the various zones represent continuous nanometric variations in stoichiometry. Zone 5b in Fig.1, for instance, and zone 9 in the same figure, however are stoechiometric. Their compositions being M₂N (zone 5b) and MN (zone 9). M may be titanium which can be replaced by a solid solution of metals of the IV B, V B or VI B elements of the periodic table of elements, in particular solid solutions of 80% of Ti with remainder being zirconium and hafnium. The solid solutions such defined will be designated as M. Such the stoechiometric composition will be M₂N and MN.

The coating of the present invention can be suitably used for deformable substrates.

The solid solid transition metal of group IV B, V B or VI B of the periodic table of the elements are Ti, V, Cr, Zr, Nb, Mo, Hf, Ta and W.

"Deformable", as used herein, refers to materials and implants whose structure can be reversibly deformed. Examples for deformable medical implants are, for instance, stents.

In a particularly preferred embodiment of the present invention the substrate material is coated with one single layer. The cross section of said single layer may be divided into various areas or stretches surrounding the substrate material having varying compositions. At least one of these areas or stretches comprises or contains M₂N, preferably Ti₂N.

The part of the single layer adjacent to the surface is composed mainly of titanium, zirconium, hafnium or alloys thereof.
This part of the single layer is responsible for the adhesion of said single layer to the surface of the substrate material. Furthermore this part may have a thickness of 1 to 5 nm, preferably 1 to 3 nm. A further area of the single layer may comprise nitrides of titanium, zirconium, hafnium or alloys thereof, such as TiN or Ti₂N more generally M₂N and MN, respectively. The titanium alloy preferably comprises 20% zirconium or hafnium. These areas or stretches may have a thickness of 2 to 30 nm, preferably 2 to 20 nm.

Those zones of the single layer which are exposed to the human or animal tissue and have to be biocompatible preferably comprise oxynitrides of titanium, zirconium, hafnium or alloys thereof. These areas or stretches may have a thickness of 2 to 30 nm, preferably 2 to 20 nm.

In order to obtain such a single layer physical vapour deposition may be used. The single layer is formed by varying the gas mixture within the device. The addition of N₂ to the inert gas (e.g. Ar) within the vapour deposition device results in the deposition of nitrides of the target metal. The addition of O₂ to the inert gas results in the formation of oxides. Adding N₂ as well as O₂ to the vacuum chamber of the vapour deposition device results in the formation of oxynitrides of the target metal.

According to a preferred embodiment of the present invention the coating layer further comprises oxides, nitrides, oxynitrides, carbides and/or borides of titanium, hafnium and/or zirconium or more generally the solid solutions of the metallic elements of group VI B, V B and VI B of the periodic table of the elements.

According to a further preferred embodiment of the present invention the oxides, nitrides, oxynitrides, carbides and/or borides are selected from the group consisting of titanium nitride (TiN), titanium oxynitride etc.

The coating layer of the present invention can be considered as "single layer coating nanostructured in stoichiometry". This means that the various zones of the coating layer have a variable, modulated stoichiometry in their cross section.

The deformable medical implant according to the present invention has to be biocompatible. In order to obtain a biocompatible surface the single layer of the present invention comprises or consists of an oxynitride, which is preferably titanium oxynitride having the formula TiNyOₓ, wherein x+y = 1, with x varying from 0.1 to 0.9, preferably from 0.2 to 0.5, particularly being 0.25.

The use of a coating layer according to the present invention has the advantage that already with one coating layer the inventive effect is achieved. Consequently, the at least one coating layer has preferably a thickness of 10 to 200 nm, preferably 20 to 100 nm, more preferably 30 to 60 nm, in particular 50 nm.

According to a preferred embodiment of the present invention an adhesion zone is provided between the substrate and the other zones of the coating layer. The adhesion zone, as discussed above, is preferably an integral part of the coating layer.

The solid transition metal of the adhesion zone is preferably zirconium, titanium, chromium or a combination thereof.

According to a preferred embodiment of the present invention the adhesion zone has a thickness of 0.5 to 15 nm, preferably 1 to 10 nm, more preferably 2 to 5 nm. The thickness of the adhesion zone depends on the energy of the impinging particles, being in the order of 30 to 200 eV.

In order to obtain a coating layer with the desired properties the zone comprising M₂N, preferably double titanium nitride (Ti₂N), has a thickness of 0.2 to 20 nm, preferably 0.5 to 15 nm, more preferably 1 to 10 nm.

According to a further preferred embodiment of the present invention the coating is comprised on substrate material selected from the group of steel, nitinol, titanium or alloys thereof as well as cobalt -titan - nickel and Co - Cr - Mo alloys.

According to a preferred embodiment of the present invention the implant is an orthopaedic implant, preferably a hip, knee, shoulder or elbow implant, or a vascular implant, preferably a vascular graft or a vascular stent.

Implants, in particular stents, being in contact with the human body or with the bloodstream should be biocompatible, in particular should exhibit anti-thrombogenic functions and favour endothehlization at the outside boundary of the stent. TiN or TiN_{y}Oₓ on the surface of the substrate or medical implant favours these properties.

Another aspect of the present invention relates to a method for producing a coating layer on a substrate using a deposition apparatus comprising a vacuum chamber and a power supply having controllable supply parameters, in which the substrate is enclosed in the vacuum chamber and subjected to bombardment by electrically-excited ions by electrical excitation provided by the power supply, said method comprising the steps of:
a) Providing an inert gas, in general Ar, and one target or several targets comprising a solid transition metal of group IV B, V B or VI B of the periodic table of the elements or a combination thereof, preferably titanium, chromium, zirconium or alloys thereof, in the vacuum chamber,
b) Performing a physical vapour deposition process for coating the surface of the substrate with an adhesion layer comprising a solid transition metal of group IV B, V B or VI B of the periodic table of the elements or a combination thereof, preferably titanium, chromium, zirconium or alloys thereof, and
c) Introducing into the vacuum chamber a gas mixture comprising an varying amount of nitrogen and/or oxygen while performing the physical vapour deposition process, wherein step c) is performed in part with a target consisting of a metal selected from a solid transition metal of group IV B, V B or VI B of the periodic table of the elements or a combination thereof, preferably titanium, zirconium or alloys thereofin an atmosphere containing 10 to 30% N₂.

The deposition apparatus according to the present invention can be anyone known in the art.

In contrast to usual methods for depositing coatings on a surface the method according to the present invention involves the use of a gas mixture whose composition changes throughout the process. This allows varying the composition of the layer during the deposition process.

Furthermore, the cross section of a layer obtainable by this method, wherein the composition of the gas mixture is changed, does not comprise interfaces between the various zones. The lack of interfaces between the zones of the coating layer is - next to the provision of at least one zone comprising double titanium nitride (Ti₂N) - responsible for the superior properties of the coating layer so that the layer on the substrate does not scale off when exposed to mechanical stress.

In order to obtain a good adhesion penetration of the zone of the coating layer adjacent to the surface of the substrate (see also zone 1 in Fig. 1) the preferred deposition methods may preferably involve high power magnetron sputtering or alternatively, for even higher particle penetration, filtered arc deposition, which can be improved by Wehnelt cylinder energy controlling techniques.

PVD procedures are well established coating procedures for electric and electronic devices. Standard sputtering and magnetron sputtering however suffer from the fact, that the primary particle energy is limited to approximatively 12 eV, thus not allowing the penetration of the sputtered particles into the substrate, with following relatively reduced adhesion, following the laws of van der Waal. This weakness was overcome, for instance, by WO 2006/067031 A1 in applying up to nine subsequent coatings of constant stoechiometry in order to compensate for internal stresses. However, as discussed above, also this solution is not satisfactory. Therefore it is preferred to apply high energy radio-frequency magnetron sputtering with resulting particle energies of up to 30 eV enabling thus ion-implantation of the sputtered ions to a depth of at least 1 nanometres (nm) as shown, for instance, in Fig. 1.

Arc deposition yields primary particle intensities of approximatively 200 eV. The ion-beam energy distribution can be uniformed by the well known Wehnelt-cylinder technique to a span of +/- 5 %. Furthermore the particles of this ion-beam, uniform in energy, can easily be accelerated to up to 1000 eV. This acceleration as well as the separation of the ions and the neutrals can be done with techniques, which are the state of the art, by electromagnetic or electrostatic fields (e.g. US 2002/007796). This variation of the particle beam intensities permit tailoring of the particle implantation into the substrate, optimizing the adhesion as a function of the substrate material and the coating material selected. The multiple coating approach can thus be essentially simplified. The addition of reactive gases in variable proportions permits the nanostructuring of the stoechiometry of the single layer in very close ranges, assuring such complete quality management of the total layer and by mastering the primary energy of the impinging particles to absolutely control the penetration depths of the aforementioned coating material and consequently completely master the critical parameter of each coating i.e. adhesion.

With the method of the present invention the modulus of Young E and the critical stress intensity factor Kic across the section of the layer can be modulated. This modulation allows to obtain a coating layer with the claimed properties.

According to a preferred embodiment of the present invention the physical vapour process is selected from the group of sputter deposition, cathodic arc deposition, reactive filtered arc plasma deposition, reactive filtered arc plasma deposition with Wehnelt plasma confinement, reactive filtered arc plasma deposition with Wehnelt plasma confinement and an electrostatic or electromagnetic scanning device and high intensity radio frequency magnetron sputtering.

In order to produce a coating as described herein the amount of nitrogen and/or oxygen in the gas mixture of step c) is stepwise or gradually increased from 0 to 30 %, preferably from 0 to 20 %, more preferably from 0 to 15 %, while performing the physical vapour deposition process to obtain a coating layer with a thickness of 10 to 200 nm, preferably 20 to 100 nm, more preferably 30 to 60 nm, in particular 50 nm.

In order to obtain the desired thickness of the various zones of the coating layer the device to perform the physical vapour process is preferably calibrated. The calibration occurs by using RBS analysis (Rutherford-Back-Scattering) which allows to determine the composition as well as the deposition rate thus obtaining a time-gas flow diagram. This diagram is used afterwards to coat the substrates of the present invention.

According to a further preferred embodiment of the present invention the amount of nitrogen in the gas mixture is stepwise or gradually increased to 30%, preferably 25%, more preferably to 20%, followed by a stepwise or gradual increase of oxygen in the gas mixture to 20%, preferably 10%.

It is particularly preferred that the ratio N₂/O₂ is about 3/1 to 5/1, preferably about 4/1.

According to a further preferred embodiment of the present invention the substrate is an implant which is preferably an orthopaedic implant, preferably a hip, knee, shoulder or elbow implant, or a vascular implant, preferably a vascular graft or a vascular stent.

According to a particularly preferred embodiment of the present invention the coating layer comprises or consists of the following cross-sectional zones (from the surface of the substrate to the surface of the coating layer): an adhesion zone having a thickness of 0.5 to 15 nm, preferably 1 to 10 nm, more preferably 2 to 5 nm, being composed of a solid transition metal of group IV B, V B or VI B of the periodic table of the elements or a combination thereof, preferably titanium, chromium and/or zirconium; at least one zone of nitrides of at least one solid transition metal of group IV B, V B or VI B of the periodic table of the elements or solid solutions thereof, preferably titanium, hafnium and/or zirconium with a thickness of 0.1 nm to 30 nm, preferably 0.5 to 20 nm; one zone comprising double titanium nitride (Ti₂N), said one zone being 0.1 to 25 nm thick; a zone comprising or consisting of titanium oxynitride has the formula TiN_{y}Oₓ, wherein x+y = 1, with x varying from 0.1 to 0.9, preferably from 0.2 to 0.5, particularly being 0.25.

Yet another aspect of the present invention relates to a coated substrate according to the present invention obtainable by a method according to the present invention.

As disclosed above the substrates to be coated with the method according to the present invention are preferably medical implants, such as stents.

An example of preferred stents are intraluminal coronary ballooning stents.

Intraluminal coronary ballooning stents represent one of the major progresses in non intrusive surgery. However, the mechanical properties of aforesaid stents have to combine a multitude of sometimes contradictory properties. On the one side they have to be very flexible in order to being introduced into the intri-cated body blood vessel system, they have to show appropriate stiffness in order to resist to buckling during stenting, during ballooning they will be submitted to a radial extension of at least 200% and at the same time will be submitted to a longitudinal shrinkage of about 10%. The ceramic coating according to the present invention may undergo a 400% radial expansion free of fissures and any scaling. Any of the claimed combinations of ceramic coatings, according to the present invention can be applied. The decision upon the right sequence nanostructured stoechiometry are physical, chemical, mechanical, biological and medical. It is important to make the right alterations between Young Modulus and critical stress intensity factor Kic, ultimate tensile strength and maximal elongation before rupture. A stent coated this way may expect a useful lifetime of 20 years. The coating would be preferentially performed by reactive filtered Arc-ion beam deposition and high power reactive radio-frequency magnetron sputtering according to the present invention.

The tribological problem of hip and knee implants is still not solved to full satisfaction. The very low coefficient of friction of titanium nitride combined with the excellent cohesion and strain-stress properties and fatigue properties of the claimed single layer coating nanostructured in its stoechiometry will provide a new solution to this important problem.

The flexible part of pacemaker electrodes can now be coated with efficient protection against Staphilococcus and bacteriophage.

Syringes for medium and long term application in the human body can be protected by the same principle. Even in case of accidental bending the protective coating will remain well positioned.

Removable electronic hearing aids, subject to multiple human intervention and thus subject to many infections, can be positively protected. All of these coatings can be performed according to the present invention and be optimized with regard to the physico-chemical requirements of the embodiments.

The coating according to the present invention may also be applied on mechanical watch regulatory mechanisms.

The regulatory mechanical watch mechanism, anchor and escape wheel, are amongst the most solicitated mechanical parts known to engineers. An important increase in lifetime or decrease in the maintenance cycle can be obtained if these mechanical parts could be coated with reliable, fatigue proof coating. A single layer coating nanostructured in its stoechiometry composed of an implantation layer of chromium and titanium followed of carbonitride of titanium or titaniumaluminide and a final stoechiometry of titanium nitride according to the present invention may be used. The anchor could be coated with a single-layer coating nanostructured in its stoechiometry of titanium or chromium or both, followed by a sandwich consisting of titaniumnitride, ti-taniumcarbide and diamond like carbon (DLC) with continous varying stoechiometry assuring thus a maintenance free timekeeping mechanism.

The present invention is further illustrated by the following figures and by the following example, however, without being restricted thereto.

Fig. 1 shows the composition of the different zones of the coating single layer in relation to the thickness of the coating layers obtainable by the method according to the present invention. M is a metal as defined herein, N is nitrogen and O is oxygen.

### EXAMPLE:

The person skilled in the art understands that the parameters given in the following example can be influenced by the parameters of the existing coating equipment, such as coating chamber geometry, pumping equipment and baffle facilities, thus influencing the residence time of the reactive species in the coating area.

A single layer coating nano-structured in its stoechiometry shall be applied to cardiovascular implants (stents).

After classical ultrasonic cleaning in appropriate aqueous solutions of cleaning agents, after drying, the stents will be placed on the substrate support. The substrate support consists of a vertical structure, submitted to a double planetary movement, allowing the stents to circulate around a central axis and suspended on individual mandrels to rotate around its own axis. 500 stents of a length of 16 mm can thus be exposed uniformly to the flux of the incoming ion-beam. Equally possible is a horizontal sample holder in case of vertical up-down coating. This sample holder would however be in a continuous oscillating horizontal movement with an amplitude of approx. 3.7 times the bare stent diameter and a frequency of approx. 1Hz. Each stent will in this case be lodged in an individual chamber according to its dimension.

The vacuum chamber was pumped to 10⁻⁷ hPa.

The chamber was filled with Ar to a pressure of 10⁻³ hPa.

The filtered and accelerated arc was ignited. During this time the substrates were protected from the ion-beam by a an appropriate shutter

After stabilisation of the ion-beam, after approx. 2 min., the shutter was opened and the substrates were subjected to a beam of 500 eV, thus creating an ion-implantation and adhesion layer of 1 to 5 nm thickness. This primary coating was performed with pure Titanium in the case of coronary implants.

Subsequently the nanometric continuous variation of the partial pressure in the coating chamber in order to achieve the desired chemical and mechanical and furthermore bioactive properties of the coating took place. During the coating of the substrate the argon arc gas was partially substituted by nitrogen by maintaining the total pressure at 10⁻³ hPa. The nitrogen partial pressure was increased continuously until reaching an intermediate zone of M₂N and later on stoichiometry of pure titanium nitride. This step of the procedure lasted approx. 5 min. In order to obtain the bioactive final surface composition it was started to replace the nitrogen by oxygen up to a proportion of 4 parts nitrogen and 1 part oxygen, which compose 10 percent of the total pressure of 10⁻³ hPa. The variation of the partial pressures of the reactive gases was done continuously or in fine steps in order to give the total coating the desired nanometric variation with regard to stoechiometry. Throughout high purity gases of grade 6.0 were used.

The same procedure applied when using high intensity reactive radio-frequency magnetron sputtering.

The coating obtainable by the method according to the present invention is further illustrated in figure 1. Zone 1 in Fig. 1 is a sub layer due to ion implantation. This layer was formed when the primary particle energy was at least 50 eV. Its thickness was between one and two nanometres (nm). This penetration layer and the subsequent layer(zone) no. 2 in Fig. 1 are essential for the good adhesion and fracture toughness of the layer, since it delivers a very high critical stress intensity factor Kic of above 200, thus stopping any potential crack and gives protection of the body tissue to potentially harmful contact with pure metal ions such as nickel, contained in any austenitic stainless steel. Zone 3 represents an intermediate zone with controlled increase of the nitrogen partial pressure in the coating reactor. The argon (Ar) partial pressure was decreased accordingly. The partial pressure of the reactive gas was in function with the reactor geometry, the metal beam (Ti, Zr or an alloy of these, but essentially titan with 20% zirconium) ionization was chosen by the person skilled in the art accordingly. Zone 4 was an intermediate stoechiometric stabilization region, with constant Kic. Zone 5a lead to the establishment of an important intermediate zone 5b, composed of stoechiometric Ti₂N (double titanium nitride), again an important barrier for crack propagation due to its mixed covalent and ionic chemical bonding. The nitrogen partial pressure was further increased in zones 6 and 8, with a relaxation zone 6 with 60% Titanium, Zirconium or its alloy of essentially 80/20 to be stabilized at 50 % nitrogen, pure titanium nitride, zone 9, known for its toughness and fatigue resistance from the tool making industry. Zone 10 follows with the introduction of oxygen in appropriate partial pressure in order to achieve the medically active layer composed of, as chosen in the presented drawing but not exclusively, 50 % titanium, 40 % nitrogen and 10 % oxygen. The titanium can be replaced by zirconium or a titanium-zirconium alloy, essentially of 80 % titanium and 20 % zirconium. More generally, but not exclusively, most solid solutions of above mentioned elements can be applied. The preferred imbodiment contains in general 80 % of titanium.

## Claims

1. Single layer ceramic coating comprising a multiplicity of cross-sectional zones, wherein the zone adjacent to the substrate is an adhesion zone composed of a solid or a combination thereof and one zone comprises double nitride (M₂N), said one zone being 0.1 to 25 nm thick; wherein M is a solid transition metal of group IV B, V B or VI B of the periodic table of the elements.

2. Coating according to claim 1, **characterised in that** the solid transition metal of the adhesion zone is zirconium, titanium, chromium or a combination thereof.

3. Coating according to claim 1 or 2, **characterised in that** M is titanium, zirconium or alloys thereof.

4. Coating according to claim 3, **characterised in that** the titanium alloy comprises 20% zirconium and/or hafnium.

5. Coating according to any one of claims 1 to 4, **characterised in that** one or more zones of the coating layer comprise oxides, nitrides, oxynitrides, carbides and/or borides of at least one solid transition metal of group IV B, V B or VI B of the periodic table of the elements.

6. Coating according to claim 5, **characterised in that** the solid transition metal of said one or more zones is titanium, chromium and/or zirconium.

7. Coating according to any one of claims 1 to 6, **characterised in that** the outermost zone of the cross-sectional zones comprises nitrides or oxynitrides of titanium, zirconium and hafnium or mixtures thereof.

8. Coating according to claim 7, **characterised in that** titanium oxynitride has the formula TiN_{y}Oₓ, wherein x+y = 1, with x varying from 0.1 to 0.9, preferably from 0.2 to 0.5, particularly being 0.25.

9. Coating according to any one of claims 1 to 8, **characterised in that** said coating layer has a thickness of 10 to 200 nm, preferably 20 to 100 nm, more preferably 30 to 60 nm, in particular 50 nm.

10. Coating according to any one of claims 1 to 9, **characterised in that** the adhesion zone has a thickness of 0.5 to 15 nm, preferably 1 to 10 nm, more preferably 2 to 5 nm.

11. Coating according to any one of claims 1 to 8, **characterised in that** the zone comprising M₂N has a thickness of 0.2 to 20 nm, preferably 0.5 to 15 nm, more preferably 1 to 10 nm.

12. Coating according to any one of claims 1 to 11, **characterised in that** the coating is comprised on substrate material selected from the group of steel, nitinol, titanium or alloys thereof.

13. Coating according to claim 12, **characterised in that** the substrate is an orthopaedic implant, preferably a hip, knee, shoulder or elbow implant, or a vascular implant, preferably a vascular graft or a vascular stent.

14. A method for producing a coating layer on a substrate using a deposition apparatus comprising a vacuum chamber and a power supply having controllable supply parameters, in which the substrate is enclosed in the vacuum chamber and subjected to bombardment by electrically-excited ions by electrical excitation provided by the power supply, said method comprising the steps of:
a) providing an inert gas and a target comprising a solid transition metal of group IV B, V B or VI B of the periodic table of the elements or a combination thereof, preferably titanium, chromium, zirconium and hafnium or alloys thereof, in the vacuum chamber,
b) performing a physical vapour deposition process for coating the surface of the substrate with an adhesion layer comprising a solid transition metal of group IV B, V B or VI B of the periodic table of the elements or a combination thereof, preferably titanium, chromium, zirconium and hafnium or alloys thereof, and
c) introducing into the vacuum chamber a gas mixture comprising an varying amount of nitrogen and/or oxygen while performing the physical vapour deposition process, wherein step c) is performed in part with a target consisting of a metal selected from a solid transition metal of group IV B, V B or VI B of the periodic table of the elements or a combination thereof, preferably titanium, zirconium or alloys thereofin an atmosphere containing 10 to 30% N₂.

15. Method according to claim 14, **characterised in that** the physical vapour process is selected from the group of sputter deposition, cathodic arc deposition, reactive filtered arc plasma deposition, reactive filtered arc plasma deposition with Wehnelt plasma confinement, reactive filtered arc plasma deposition with Wehnelt plasma confinement and an electrostatic or electromagnetic scanning device and high intensity radio frequency magnetron sputtering.

16. Method according to claim 14 or 15, **characterised in that** the amount of nitrogen and/or oxygen in the gas mixture of step c) is stepwise or gradually increased from 0 to 30%, preferably from 0 to 20%, more preferably from 0 to 15%, while performing the physical vapour deposition process to obtain a coating layer with a thickness of 10 to 200 nm, preferably 20 to 100 nm, more preferably 30 to 60 nm, in particular 50 nm.

17. Method according to any one of claims 14 to 15, **characterised in that** the amount of nitrogen in the gas mixture is stepwise or gradually increased to 30%, preferably 25%, more preferably to 20%, followed by an stepwise or gradual increase of oxygen in the gas mixture to 20%, preferably 10%.

18. Method according to any one of claims 14 to 17, **characterised in that** the substrate is an implant which is preferably an orthopaedic implant, preferably a hip, knee, shoulder or elbow implant, or a vascular implant, preferably a vascular graft or a vascular stent.

19. Substrate comprising a coating according to any one of claims 1 to 13 obtainable by the method according to any one of claims 14 to 18.
